# EUROPEAN PATENT APPLICATION

(11) **EP 4 691 369 A1**
(43) Date of publication of application: **11.02.2026**
(21) Application number: 24781328.0
(22) Date of filing: 29.03.2024
(51) Int. Cl.: A61B 5/361, A61B 5/00, A61B 6/00, G16H 50/20, G16H 50/30, G16H 50/50

(54) **ARTIFICIAL INTELLIGENCE-BASED HEART DISEASE RISK PREDICTION DEVICE AND METHOD**

(30) Priority: 30.03.2023 KR 20230041988
(71) Applicant: Seoul National University Hospital, Seoul 03080 (KR)
(72) Inventor: KIM, Joonghee, Seongnam-si, Gyeonggi-do 13620 (KR); CHO, Youngjin, Seongnam-si, Gyeonggi-do 13620 (KR); LEE, Jihyun, Seongnam-si, Gyeonggi-do 13620 (KR)
(74) Representative: V.O.
(86) International application number: PCT/KR2024/004112
(87) International publication number: WO 2024/205338

(57) **Abstract**

Disclosed are an artificial intelligence-based heart disease risk prediction device and method, according to one embodiment. The artificial intelligence-based heart disease risk prediction device, according to one embodiment, is provided with: one or more processors; and one or more memories for storing instructions executed by the one or more processors, wherein the one or more processors: input at least one of first input data and second input data of a subject into a trained model; and predict the risk of heart disease of the subject based on a result output by the trained model.

## Description

### Technical Field

The disclosed embodiments relate to an artificial intelligence-based heart disease risk prediction device and method, and more particularly, to a technique for indicating the risk of developing paroxysmal atrial fibrillation or new on-set atrial fibrillation in the near future on the basis of a result obtained by using an electrocardiogram and/or chest X-ray.

### [Cross-reference to related applications]

This application claims priority to Korean Provisional Patent Application No. 10-2023-0041988, filed on March 30, 2023, the disclosure of which is incorporated herein in its entirety.

### Background Art

A deep learning model based on an artificial neural network structure may be trained to evaluate the risk of various heart diseases by analyzing digital time series data obtained by digitizing a waveform of an electrocardiogram or image data obtained by converting the digital time series data into an image. Therefore, a technology for applying such an artificial intelligence system to various heart disease areas has been developed in recent years.

### Detailed Description of the Invention

### Technical Solution

A heart disease, such as paroxysmal atrial fibrillation, is a disease in which episodes of atrial fibrillation occur intermittently and usually stop on its own. Thus, if a monitoring operation is not performed with an electrocardiogram device for a long period of time, the paroxysmal atrial fibrillation may be missed. However, such a monitoring operation is expensive and inconvenient since patients have to wear monitoring equipment for a long period of time.

In recent years, many small, attachable electrocardiogram devices have been developed and sold to alleviate these difficulties. However, these devices are also expensive and must be attached to the body for a long period of time, which sometimes causes serious skin allergies. Thus, it is difficult to apply this monitoring operation to all patients. As a result, a separate high-risk screening method is needed that allows the monitoring operation to be selectively applied only to high-risk patients.

The disclosed embodiments provide an artificial intelligence-based heart disease risk prediction device and method to locate a high risk group for heart disease, such as paroxysmal atrial fibrillation, by utilizing input information, such as chest X-ray and electrocardiogram (one-time, non-monitoring) that can be performed for a short period of time, e.g., within one minute, without long-term electrocardiogram monitoring.

### Summary of the Invention

An artificial intelligence-based heart disease risk prediction device, according to an embodiment, is provided with one or more processors, and one or more memories for storing instructions executed by the one or more processors, wherein the one or more processors input at least one among first input data and second input data of a subject into a trained model, and predict the risk of heart disease of the subject based on a result output by the trained model.

The trained model includes an encoder unit configured to convert input data including at least one of the first input data and the second input data into a vector, and a task unit configured to assign a score to the input data to predict a probability that the subject has heart disease.

The task unit may be configured to calculate a corrected score by reflecting additional information about the subject to a raw score for the input data, and predict a probability that the subject has heart disease based on the corrected score.

The additional information may be information input by the subject or generated by preprocessing pre-stored record information by the encoder unit.

The task unit may include a first task unit configured to calculate a corrected first individual score by reflecting the additional information to a first vector for the first input data; and a second task unit configured to calculate a corrected second individual score by reflecting the additional information to a second vector for the second input data.

The task unit may be configured to calculate a corrected integrated score by reflecting the additional information to an entire vector generated based on the first vector for the first input data and the second vector for the second input data.

The trained model may be trained based on constructed learning data, and the learning data may be constructed by labeling the obtained input data with any one of a first label, a second label, and a pseudo-label.

The learning data may be constructed by labeling the input data with the first label when heart disease in the medical history of the patient that is a source of the obtained input data is already diagnosed at a first time point, and labeling the input data with the first label when an abnormal symptom corresponding to heart disease in the obtained input data is detected at a second time point.

The learning data may be constructed by labeling the input data with the pseudo-label when the obtained input data is censored and measured before the second time point and no abnormal symptom is detected in the input data until a third time point before the second time point.

The learning data may be constructed by labeling the input data with the second label when the obtained input data is measured until a fourth time point after the second time point and no abnormal symptom is detected in the input data until the fourth time point.

The first input data may be electrocardiogram information, the second input data may be chest X-ray information, and heart disease may include atrial fibrillation.

An artificial intelligence-based heart disease risk prediction method, according to an embodiment, is a method performed by the artificial intelligence-based heart disease risk prediction device including one or more processors and one or more memories for storing instructions executed by the one or more processors, the method including inputting at least one of first input data and second input data of a subject into a trained model, and predicting the risk of heart disease of the subject based on a result output by the trained model.

The trained model includes an encoder unit configured to convert input data including at least one of the first input data and the second input data into a vector, and a task unit configured to assign a score to the input data to predict a probability that the subject has heart disease.

The task unit may be configured to calculate a corrected score by reflecting additional information about the subject and adjusting a raw score for the input data, and predict a probability that the subject has heart disease based on the corrected score.

The additional information may be information input by the subject or generated by preprocessing pre-stored record information by the encoder unit.

The task unit may include a first task unit configured to calculate a corrected first individual score by reflecting the additional information to a first vector for the first input data, and a second task unit configured to calculate a corrected second individual score by reflecting the additional information to a second vector for the second input data.

The task unit may be configured to calculate a corrected integrated score by reflecting the additional information to an entire vector generated based on the first vector for the first input data and the second vector for the second input data.

The trained model may be trained based on constructed learning data, and the learning data may be constructed by labeling the obtained input data with any one of a first label, a second label, and a pseudo-label.

The learning data may be constructed by labeling the input data with the first label when heart disease in the medical history of the patient that is a source of the obtained input data is already diagnosed at a first time point and by labelling the input data with the first label when an abnormal symptom corresponding to heart disease in the obtained input data is detected at a second time point.

The learning data may be constructed by labeling the input data with the pseudo-label when the obtained input data is censored and measured before the second time point and no abnormal symptom is detected in the input data until a third time point before the second time point.

The learning data may be constructed by labeling the input data with the second label when the obtained input data is measured until a fourth time point after the second time point and no abnormal symptom is detected in the input data until the fourth time point.

The first input data may be electrocardiogram information, the second input data may be chest X-ray information, and heart disease may include atrial fibrillation.

### Effects of Invention

According to the disclosed embodiments, when a high-risk group of heart disease, such as paroxysmal atrial fibrillation, is selected, electrocardiogram monitoring can be selectively applied, thereby reducing medical costs and improving patient convenience.

In addition, according to the disclosed embodiments, it is possible to construct insufficient medical data as learning data through pseudo-labeling or the like, and thus it is possible to efficiently learn a trained model in an environment where input data is limited.

### Brief Description of the Drawings

FIG. 1 is a block diagram of an artificial intelligence-based heart disease risk prediction device, according to an embodiment.
FIG. 2 is a diagram of an architecture of a trained model according to an embodiment.
FIG. 3 is a diagram illustrating an example of an inference process performed by the trained model of FIG. 2.
FIG. 4 is a diagram illustrating another example of the inference process performed by the trained model of FIG. 2.
FIG. 5 is an illustrative diagram of the architecture of an additional trained model and the inference process in one embodiment.
FIG. 6 is an illustrative diagram of the architecture of an additional trained model and the inference process in one embodiment.
FIG. 7 is an illustrative diagram of the architecture of an additional trained model and the inference process in one embodiment.
FIG. 8 is a diagram illustrating an algorithm for constructing learning data according to an embodiment.
FIG. 9 is a flowchart illustrating an artificial intelligence-based heart disease risk prediction method according to an embodiment.

### Best Mode for Implementing the Invention

The following description is provided to provide a comprehensive understanding of a method, a device, and/or a system described herein. However, this is merely an example and the present disclosure is not limited thereto.

In describing the embodiments, detailed descriptions of well-known technologies related to the present disclosure will be omitted when it is determined that the detailed descriptions may unnecessarily obscure the gist of the embodiments.

The terms described below are terms defined in consideration of functions in the present invention, which may vary according to the intention or practice of a user, an operator, or the like. Therefore, the definition should be based on the content throughout the specification. The terms used in the detailed description are only for illustrating one embodiment but not for limiting the same.

Unless explicitly used otherwise, the singular forms include plural forms. The first, second, and the like are used for distinguishing various elements only and are not limited by the terms.

The term "heart disease" as used herein includes atrial fibrillation.

The term "trained model" as used herein refers to a trained model that predicts the risk of heart disease, such as atrial fibrillation.

The term "atrial fibrillation" as used herein includes atrial fibrillation, or atrial fibrillation and atrial flutter.

The device of the present invention may be entirely hardware, or partially hardware and partially software. For example, a unit may collectively refer to a device for transmitting and receiving data of a specific format and content by an electronic communication method, and software related thereto.

Herein, terms such as "unit", "module", "server", "system", "device", or "terminal" (e.g., an encoder unit or a task unit) are intended to refer to a combination of hardware and software driven by the hardware. For example, the hardware may be a data processing device including a CPU or another processor. In addition, software driven by hardware may refer to an executing process, an object, an executable, a thread of execution, a program, or the like.

The encoder unit herein may also be referred to as an encoder or an encoding performing processor. The task unit may likewise be referred to as a task performing processor.

Herein, the term "before" is referred to as being able to include both the present and the past.

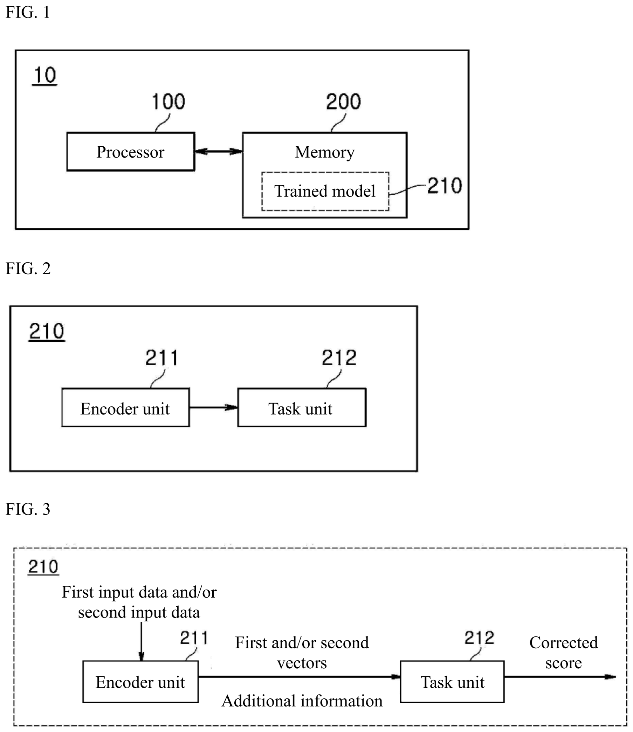

FIG. 1 is a block diagram of an artificial intelligence-based heart disease risk prediction device 10 according to an embodiment.

Referring to FIG. 1, the artificial intelligence-based heart disease risk prediction device 10 includes a processor 100 and a memory 200.

The processor 100 inputs at least one of first input data and second input data of a subject to a trained model 210.

The first input data and the second input data may be information indicating an electrical activity of the heart and information indicating an anatomy, respectively. For example, the first input data and the second input data may be electrocardiogram information and chest X-ray information, respectively. Alternatively, the first input data and the second input data may be chest X-ray information and electrocardiogram information, respectively.

In a non-limiting example, the electrocardiogram information, which is multi-channel electrocardiogram information, may include, for example, electrocardiogram information measured from a plurality of leads (e.g., 12-lead electrocardiogram) and may include multi-channel one-dimensional time series data. Alternatively, the electrocardiogram information may be an image data array obtained by converting each time series data into an image in one two-dimensional plane. Alternatively, the electrocardiogram signal may be image array data obtained by capturing or photographing the image data array. That is, the electrocardiogram information is intended to have a free form regardless of the data form as long as it is information recording the electrical activity of the heart.

In a non-limiting example, the second input data may be chest X-ray information generated from an X-ray imaging device. The chest X-ray information, which is a radiography image taken from various directions, may preferably include a data array or an original image having a standard format. For example, the chest X-ray information may be a medical digital image and a communication standard (DICOM) image original file or an image data array taken from radiation radiated from the back to the front (PA), from the front to the back (AP), and from one side to the other side (LAT) of the subject, or may be a chest X-ray film or an image data array obtained by capturing or photographing the DICOM image. That is, the chest X-ray information is also intended to have a free format regardless of the data form as long as it is information recording the anatomy of the heart.

The processor 100 predicts heart disease of the subject based on the risk prediction result output by the trained model 210.

The heart disease may typically include paroxysmal atrial fibrillation. As a specific example, heart disease, which is a variety of types of atrial fibrillation classified according to expression pattern, duration, and spontaneous termination, may include paroxysmal and permanent atrial fibrillation. In addition, as described above, heart disease may further include atrial flutter.

The processor 100 may predict the risk of heart disease, such as paroxysmal atrial fibrillation of the subject, based on a score calculated by analyzing at least one of the first input data and the second input data by the trained model 210. That is, the processor 100 may predict the risk of heart disease of the subject based on a result obtained by analyzing the first input data, the second input data, or the entire information obtained by integrating the first input data with the second input data.

The processor 100 may predict the risk of heart disease of the subject based on a corrected score calculated by further adding additional information to at least one of the first input data and the second input data by the trained model 210. For example, the processor 100 may predict the risk of heart disease of the subject based on a corrected score calculated by further adding additional information to the first input data, the second input data, or the entire information obtained by integrating the first input data with the second input data by the training model 210.

The additional information, which is information on the health status of the subject, may include demographic information, symptoms, signs, disease history, and other test results of the subject.

As a specific example, the additional information may include a value related to at least one of the subject's age, gender, symptoms such as palpitations, underlying diseases, such as heart failure, vital signs, echocardiographic measurements, and Halter measurements. The additional information may be a numerical vector obtained by transforming the above-mentioned information using normalization or embedding and extracting the information using a separate artificial intelligence encoder (preprocessing of the additional information).

The memory 200 stores one or more instructions executed by the processor 100. The memory 200 may store the trained model 210 accessed by the processor 100. The architecture of the trained model 210 and the inference process will be described below.

Although the trained model 210 is illustrated as being stored in the memory 200 in FIG. 1, the trained model 210 may be stored in an external storage device, unlike the illustrated example, and is not necessarily limited to the illustrated example.

FIG. 2 is a diagram of the architecture of the trained model 210.

Referring to FIG. 2, the trained model 210 includes an encoder unit 211 and a task unit 212.

The encoder unit 211 may be configured to generate a vector corresponding to input data of the trained model 210. The input data may include first input data and second input data.

For example, the encoder unit 211 may extract a feature from the input data in the form of an image and convert the image into a vector.

As another example, the encoder unit 211 may extract a feature from the multi-channel time series input data and convert the time series data into a vector.

As another example, the encoder unit 211 may convert a word or sentence(s) into a vector by performing word or document embedding on the input data in a text format.

The encoder unit 211 may include at least one of a convolutional neural network, a recurrent neural network, a transformer, and a multi-layer perceptron. In this case, the neural network of the encoder unit 211, which is already optimized or trained for other uses, is preferably utilized, but is not limited thereto.

The task unit 212 may be configured to assign a score to the input data to predict a probability that the subject has heart disease.

The task unit 212 may be configured to predict the probability of heart disease by using a vector for the input data output by the encoder unit 211 (i.e., an output vector of the encoder unit) as an input value. In addition, the task unit 212 may further include a numerical vector corresponding to the additional information as an additional input value.

The task unit 212 may be configured as a classifier that includes a fully-connected layer and an activation function, e.g., passing the final output through a Sigmoid function to output a value between 0 and 1 as a probability.

The encoder unit 211 and the task unit 212 may be trained using an optimizer (e.g., stochastic gradient descent, Nesterov accelerated gradient, RMSprop, Adam, Adam-W optimizer) and a loss function (e.g., cross-entropy loss with/without label 0smoothing).

FIG. 3 is a diagram of an example of the inference process performed by the trained model 210.

The encoder unit 211 of the trained model 210 may map the input data to a low-dimensional space to generate a corresponding vector. For example, when the encoder unit 212 receives the first input data and/or the second input data as the input data, the encoder unit 211 may generate a corresponding first vector and/or second vector. In other words, when the encoder unit 211 receives only the first input data, only the first vector may be output, and when the encoder unit 211 receive the first input data and the second input data, the first vector and the second vector corresponding to the first input data may be output.

At this time, the task unit 212 may add the first vector and the second vector as well as the additional information to calculate a corrected score in relation to heart disease retention, such as paroxysmal atrial fibrillation.

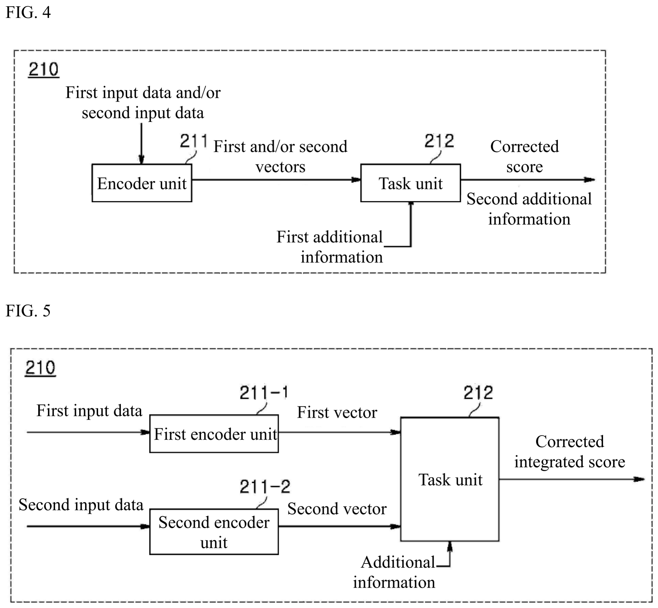

Another example of the inference process of the trained model 210 will now be described with reference to FIG. 4.

FIG. 4 is a diagram of another example of the inference process performed by the trained model 210. For convenience of description, redundant descriptions will be omitted.

The task unit 212 of the trained model 210 may calculate a corrected score related to heart disease based on the first vector, the second vector, and first additional information.

For example, the task unit 212 may calculate a raw score related to heart disease based on the first vector and the second vector, and calculate a corrected integrated score related to heart disease by reflecting the first additional information to the raw score.

Thereafter, the task unit 212 may further calculate a corrected final integrated score by reflecting the second additional information to the corrected integrated score. In this case, the first and second additional information may be provided in a preprocessed form to enable the trained model 210 to process the first and second information.

FIG. 5 is an illustrative diagram of the architecture of an additional trained model 210 and the inference process in one embodiment. For convenience of description, redundant descriptions will be omitted.

Referring to FIG. 5, the additional trained model 210 may include a first encoder unit 211-1, a second encoder unit 211-2, and a task unit 212. The first encoder unit 211-1 and the second encoder unit 211-2 may be used separately to effectively process different types of input data.

The first encoder unit 211-1 may be used to convert time-series data into a vector. For example, the first encoder unit 211-1 may be configured to receive, as first input data, electrocardiogram data as an input to generate a corresponding vector.

The second encoder unit 211-2 may be used to convert the image data into a vector. For example, the second encoder unit 211-2 may be configured to accept, as the second input data, the chest X-ray data as an input to generate a corresponding vector.

The first and the second are referred to to identify different entities from each other and are not limited to the above-described example. That is, the first encoder unit 211-1 and the second encoder unit 211-2 may perform reverse functions, unlike the above-described examples.

The task unit 212 may receive the first vector and the second vector output from the first encoder unit 211-1 and the second encoder unit 212-2, respectively, and may calculate the corrected integrated score by using the additionally received additional information. In this case, the task unit 212 may calculate the integrated score based on an entire vector obtained by integrating the first vector with the second vector and may calculate a corrected integrated score by reflecting the additional information.

In FIG. 5, the trained model 210 is preferably used when both the first input data and the second input data are obtained because different types of input data are processed.

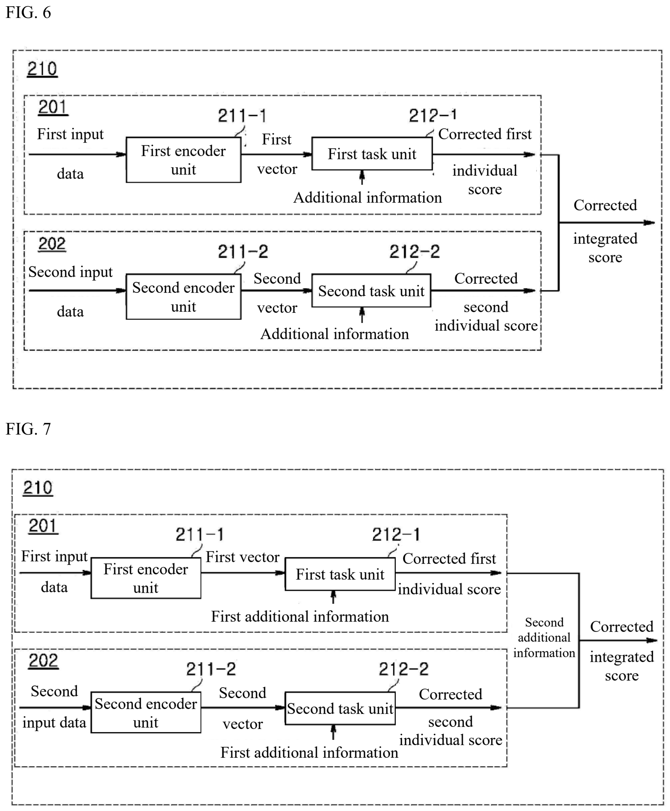

FIG. 6 is an illustrative diagram of the architecture of an additional trained model 210 and the inference process. For convenience of description, redundant descriptions will be omitted.

The additional trained model 210 of FIG. 6 may include a first trained model 201 and a second trained model 202.

The first trained model 201 may be a model configured to calculate a score corresponding to the first input data. The second trained model 202 may be a model configured to calculate a score corresponding to the second input data.

Specifically, the first trained model 201 may encode first input data in the first encoder unit 211-1 to generate a first vector and may receive the first vector and additional information as inputs in the first task unit 212-1 to calculate a corrected first individual score.

Similarly, the second trained model 202 may encode second input data in the second encoder unit 211-2 to generate a second vector and may receive the second vector and additional information as inputs in the second task unit 212-2 to calculate a corrected second individual score.

Then, the trained model 210 may calculate a corrected integrated score based on the corrected first individual score and the corrected second individual score output by the first trained model 201 and the second trained model 202, respectively.

Specifically, the trained model 210 may calculate the corrected integrated score based on an arithmetic mean or a weighted mean for the corrected first individual score and the corrected second individual score.

The trained model 210 may use the corrected first individual score and the corrected second individual score as input data to calculate a corrected integrated score as an output result obtained when applying a regression analysis, a machine learning algorithm, or an artificial neural network model.

In FIG. 6, the trained model 210 is preferably used when both the first input data and the second input data are obtained because different types of input data are processed.

FIG. 7 is an illustrative diagram of the architecture of an additional trained model 210 and the inference process.

The additional trained model 210 of FIG. 7 may include a first trained model 201 and a second trained model 202 as shown in FIG. 6.

The first additional information may be used to correct the individual score, the first vector, and the second vector in the second encoder unit 211-2, the first task unit 212-1, and the second task unit 212-2, respectively. The second additional information may also be used to correct an integrated score.

In FIG. 7, the trained model 210 is preferably used when both the first input data and the second input data are obtained because different types of input data are processed.

As illustrated and described with reference to FIGS. 2 to 7, the trained model 210 may have an architecture in which the number and combination of encoder units 211 and task units 212 vary, and the additional information may be input to an input end of the task unit 212 as a numerical vector or may be further added to an output value of the task unit 212.

In addition, the encoder unit 211, the task unit 212, the first encoder unit 211-1, the second encoder unit 211-2, the first task unit 212-1, and the second task unit 212-2 illustrated and described with reference to FIGS. 2 to 7 may be individually trained according to a type of input data to be processed. For example, it is preferable that the encoder unit 210 and the task unit 212 are trained based on a pair of first input data and second input data, and the first encoder units 210-1 and 210-2 are trained based on the first input data or the second input data, respectively.

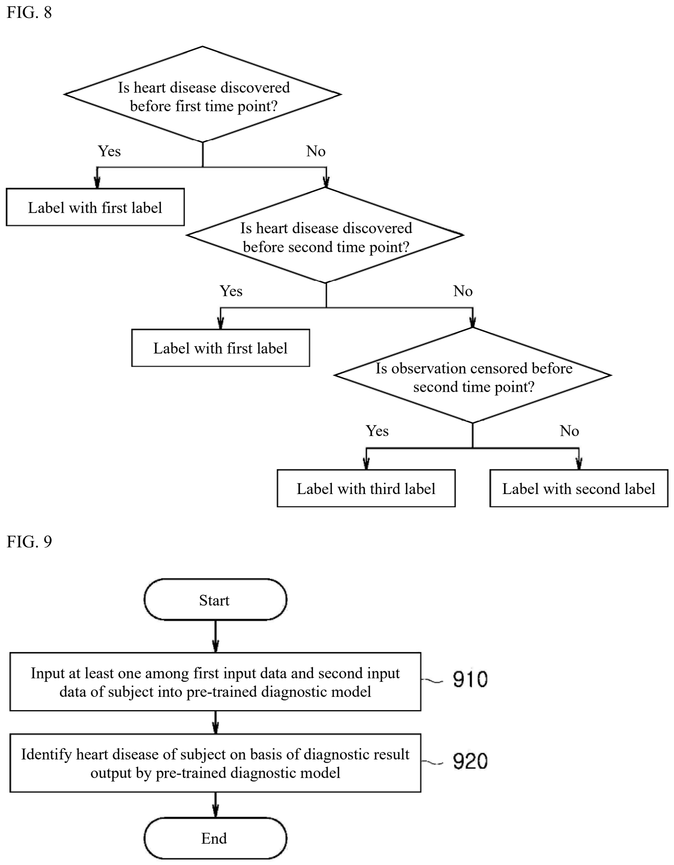

FIG. 8 is a diagram illustrating an algorithm for constructing learning data.

Referring to FIG. 8, a labeling classification scheme has a hierarchical structure configured to assign labels to data using medical results at particular points in time.

First, when the patient which is a source of the input data is confirmed to have heart disease before a first time point, the input data may be labeled with a first label. The first time point may be a time point at or before obtaining the input data.

For example, when a medical record, pre-measured vital sign information, and a pre-analyzed diagnostic result for the patient who provided the input data indicate the presence of heart disease, the input data may be labeled with the first label and constructed as learning data.

The first label may mean positive.

The medical record may record the presence or absence of atrial fibrillation, the time when the atrial fibrillation was diagnosed, the input time point, and the like. The presence or absence of the atrial fibrillation and the test time point may be recorded in the pre-measured vital sign information or may be calculated through a separate algorithm. The pre-analyzed diagnosis result may record the presence or absence and the time point of the atrial fibrillation detected during the monitoring in the Holter test, the loop recorder, and the wearable medical device.

Secondly, when the input data is confirmed to include an abnormal symptom due to heart disease before the second time point, the input data may be labeled with the first label. The second time point may be a time point after the first time point, for example, a preset time point at which monitoring of the input data is to be terminated.

In a specific example, when input data for a patient who does not have heart disease is obtained and an abnormal symptom due to heart disease is found in the input data, the input data may be labeled with the first label and may be constructed as learning data.

Any abnormal symptoms may then be detected using a separate trained model.

Thirdly, when the obtained input data is censored before the second time point and is measured incompletely and no abnormal symptom is detected in the input data until a third time point before the second timepoint, the input data may be labeled with a third label.

The term "censoring" may refer to a state in which observation is stopped without finding abnormal symptoms of heartbeat before the originally planned observation period.

The third label may refer to a pseudo-label. The pseudo-label, which is a value predicted by the trained model 210 with respect to corresponding data, may mean, for example, a probability of having heart disease, such as paroxysmal atrial fibrillation.

The third time point may be a time point after the first time point at which the input data starts to be measured and before the second time point corresponding to the time point at which pre-planned measurement for the input data ends.

When the obtained input data is not censored before the second time point, that is, completely measured until a fourth time point after the second time point and no abnormal symptom is detected in the input data until the fourth time point, the input data may be labeled with the second label.

The second label may mean negative.

The measurement of the input data and the determination of the abnormal symptom may be initiated or determined by medical examination, such as electrocardiogram, Halter, loop recorder, etc.

The trained model 210 may be semi-supervised by the constructed learning data in FIG. 8 and may be supervised by other pre-constructed learning data.

The learning data may be constructed by the processor 100 or an external entity through the classification system of FIG. 8.

FIG. 9 is a flowchart illustrating an artificial intelligence-based heart disease risk prediction method, according to an embodiment.

Referring to FIG. 9, the method of FIG. 9 may be performed by the artificial intelligence-based heart disease risk prediction device 10 according to an embodiment of FIG. 1.

First, the artificial intelligence-based heart disease risk prediction device 10 according to an embodiment inputs at least one of first input data and second input data of the subject into the trained model 210 (910).

Then, the artificial intelligence-based heart disease risk prediction device 10 according to an embodiment predicts the risk of heart disease of the subject based on a result output by the trained model 210 (920).

Although FIG. 9 describes the method with a plurality of steps, at least some of the steps may be performed in a reverse order, may be combined with other steps and performed together, may be omitted, may be divided into detailed steps, or may be performed by adding one or more steps that are not illustrated herein.

While the exemplary embodiments of the present invention have been described in detail above, those skilled in the art will understand that various modifications can be made to the above-described embodiments without departing from the scope of the present invention. Therefore, the scope of the invention should not be limited to the described embodiments but should be defined by the following claims and their equivalents.

### Industrial Applicability

The artificial intelligence-based heart disease risk prediction device and method according to an embodiment are applicable to the digital medical industry since they predict the risk of heart disease using a trained model.

## Claims

1. An artificial intelligence-based heart disease risk prediction device comprising:
one or more processors; and
one or more memories for storing instructions executed by the one or more processors, wherein the one or more processors are configured to input at least one of first input data and second input data of a subject into a trained model, and predict the risk of heart disease of the subject based on a result output by the trained model.

2. The artificial intelligence-based heart disease risk prediction device of claim 1, wherein the trained model includes an encoder unit configured to convert input data including at least one of the first input data and the second input data into a vector, and a task unit configured to assign a score to the input data to predict a probability that the subject has heart disease.

3. The artificial intelligence-based heart disease risk prediction device of claim 2, wherein the task unit is configured to calculate a corrected score by reflecting additional information about the subject to a raw score for the input data, and predict a probability that the subject has heart disease based on the corrected score.

4. The artificial intelligence-based heart disease risk prediction device of claim 3, wherein the additional information is information input by the subject or generated by preprocessing pre-stored record information by the encoder unit.

5. The artificial intelligence-based heart disease risk prediction device of claim 3, wherein the task unit comprises a first task unit configured to calculate a corrected first individual score by reflecting the additional information to a first vector for the first input data, and a second task unit configured to calculate a corrected second individual score by reflecting the additional information to a second vector for the second input data.

6. The artificial intelligence-based heart disease risk prediction device of claim 3, wherein the task unit is configured to calculate a corrected integrated score by reflecting the additional information to an entire vector generated based on the first vector for the first input data and the second vector for the second input data.

7. The artificial intelligence-based heart disease risk prediction device of claim 1, wherein the trained model is trained based on constructed learning data, and the learning data is constructed by labeling the obtained input data with any one of a first label, a second label, and a pseudo-label.

8. The artificial intelligence-based heart disease risk prediction device of claim 7, wherein the learning data is constructed by labeling the input data with the first label when heart disease in the medical history of the patient that is a source of the obtained input data is already diagnosed at a first time point, and labeling the input data with the first label when an abnormal symptom corresponding to heart disease in the obtained input data is detected at a second time point.

9. The artificial intelligence-based heart disease risk prediction device of claim 7, wherein the learning data is constructed by labeling the input data with the pseudo-label when the obtained input data is censored and measured before a second time point and no abnormal symptom is detected in the input data until a third time point before the second time point.

10. The artificial intelligence-based heart disease risk prediction device of claim 7, wherein the learning data is constructed by labeling the input data with the second label when the obtained input data is measured until a fourth time point after the second time point and no abnormal symptom is detected in the input data until the fourth time point.

11. The artificial intelligence-based heart disease risk prediction device of any one of claims 1 to 10, wherein the first input data is electrocardiogram test information, the second input data is chest X-ray information, and heart disease comprises atrial fibrillation.

12. An artificial intelligence-based heart disease risk prediction method performed by an artificial intelligence-based heart disease risk prediction device having one or more processors; and one or more memories for storing instructions executed by the one or more processors, the method comprising:
inputting at least one of first input data and second input data of a subject into a trained model; and
predicting the risk of heart disease of the subject based on a result output by the trained model.

13. The artificial intelligence-based heart disease risk prediction method of claim 12, wherein the trained model includes an encoder unit configured to convert input data including at least one of the first input data and the second input data into a vector; and a task unit configured to assign a score to the input data to predict a probability that the subject has heart disease.

14. The artificial intelligence-based heart disease risk prediction method of claim 13, wherein the task unit is configured to calculate a corrected score by reflecting additional information about the subject and adjusting a raw score of the input data, and predict a probability that the subject has heart disease based on the corrected score.

15. The artificial intelligence-based heart disease risk prediction method of claim 14, wherein the additional information is information input by the subject or generated by preprocessing pre-stored record information by the encoder unit.

16. The artificial intelligence-based heart disease risk prediction method of claim 14, wherein the task unit comprises a first task unit configured to calculate a corrected first individual score by reflecting the additional information to a first vector for the first input data; and a second task unit configured to calculate a corrected second individual score by reflecting the additional information to a second vector for the second input data.

17. The artificial intelligence-based heart disease risk prediction method of claim 14, wherein the task unit is configured to calculate a corrected integrated score by reflecting the additional information to an entire vector generated based on the first vector for the first input data and the second vector for the second input data.

18. The artificial intelligence-based heart disease risk prediction method of claim 12, wherein the trained model is trained based on the constructed learning data, and the learning data is constructed by labeling the obtained input data with any one of a first label, a second label, and a pseudo-label.

19. The artificial intelligence-based heart disease risk prediction method of claim 18, wherein the learning data is constructed by labeling the input data with the first label when heart disease in the medical history of the patient which is a source of the obtained input data is diagnosed at a first time point and by labeling the input data with the first label when an abnormal symptom corresponding to heart disease in the obtained input data is detected at a second time point.

20. The artificial intelligence-based heart disease risk prediction method of claim 18, wherein the learning data is constructed by labeling the input data with the pseudo-label when the obtained input data is censored and measured before a second time point and no abnormal symptom is detected in the input data until a third time point before the second time point.

21. The artificial intelligence-based heart disease risk prediction method of claim 18, wherein the learning data is constructed by labeling the input data with the second label when the obtained input data is measured until a fourth time point after the second time point and no abnormal symptom is detected in the input data until the fourth time point.

22. The artificial intelligence-based heart disease risk prediction method of any one of claims 12 to 21, wherein the first input data is electrocardiogram test information, the second input data is chest X-ray information, and heart disease comprises atrial fibrillation.
